(19) Europäisches Patentamt
European Patent Office
Office européen des brevets

(11) Veröffentlichungsnummer: **0 286 578 A1**

(12) **EUROPÄISCHE PATENTANMELDUNG**

(21) Anmeldenummer: **88730076.2**

(22) Anmeldetag: **25.03.88**

(51) Int. Cl.4: **C 07 J 1/00**
C 07 J 41/00, A 61 K 31/565

(30) Priorität: **08.04.87 DE 3711772**

(43) Veröffentlichungstag der Anmeldung:
**12.10.88 Patentblatt 88/41**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Bohlmann, Rolf, Dr.**
**Melanchtonstrasse 23**
**D-1000 Berlin 21 (DE)**

**Henderson, David, Dr.**
**Jahnstrasse 17**
**D-1000 Berlin 28 (DE)**

**Kerb, Ulrich, Dr.**
**Prinzregentenstrasse 7**
**D-1000 Berlin 31 (DE)**

**Nishino, Yukishige, Dr.**
**Lanzendorfer Weg 14**
**D-1000 Berlin 22 (DE)**

**Sauer, Gerhard, Dr.**
**Königsbacher Zeile 41 A**
**D-1000 Berlin 28 (DE)**

(54) **4,15-Disubstituierte 4-Androsten-3,17-dione und Verfahren zu deren Herstellung.**

(57) 1.) 4,15-Disubstituierte 4-Androsten-3,17-dione der allgemeinen Formel I

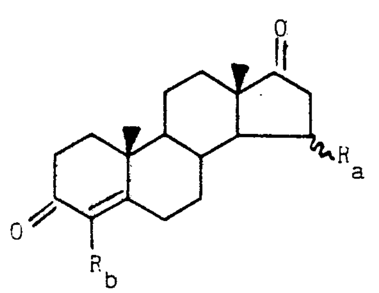

(I) ,

worin
$R_a$ einen gesättigten oder ungesättigten, gegebenenfalls substituierten Alkylrest mit 1-10 Kohlenstoffatomen, wobei $R_a$ in der α- oder β-Position steht,
$R_b$ ein Halogen, eine Azido-, eine $-OR_1$ oder $-NR_2R_3$ Gruppe, wobei $R_1$ ein Wasserstoffatom oder eine Acylgruppe mit 1-10 Kohlenstoffatomen und $R_2$ und $R_3$ gleich oder verschieden sind und jeweils ein Wasserstoffatom, eine Alkyl-oder Acylgruppe mit jeweils 1-10 Kohlenstoffatomen darstellen, bedeuten.

Die neuen Verbindungen der allgemeinen Formel 1 sind zur Fertilitätskontrolle und zur Behandlung von Krankheiten geeignet, die durch Östrogene hervorgerufen werden.

EP 0 286 578 A1

## Beschreibung

### 4,15-Disubstituierte 4-Androsten-3,17-dione und Verfahren zu deren Herstellung

Die Erfindung betrifft 4,15-disubstituierte 4-Androsten-3,17-dione, Verfahren zu deren Herstellung und diese enthaltende pharmazeutische Präparate.

Die erfindungsgemäßen Verbindungen werden durch die allgemeine Formel I beschrieben

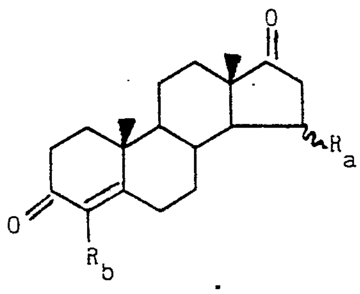

(I) ,

worin

$R_a$ einen gesättigten oder ungesättigten, gegebenenfalls substituierten Alkylrest mit 1-10 Kohlenstoffatomen, wobei $R_a$ in der α- oder β-Position steht,

$R_b$ ein Halogen, eine Azido-, eine $-OR_1$ oder $-NR_2R_3$-Gruppe, wobei $R_1$ ein Wasserstoffatom oder eine Acylgruppe mit 1-10 Kohlenstoffatomen und $R_2$ und $R_3$ gleich oder verschieden sind und jeweils ein Wasserstoffatom, eine Alkyl-oder Acylgruppe mit jeweils 1-10 Kohlenstoffatomen darstellen, bedeuten.

Die für $R_a$ stehenden Alkylgruppen sollen gerad- oder verzweigtkettig, gesättigt oder ungesättigt, substituiert oder unsubstituiert sein und 1-10 Kohlenstoffatome enthalten. Diese Alkylgruppen können am Steroidgerüst α- oder β-ständig sein. Bei einer substituierten Alkylgruppe können sich bis zu fünf Substituenten an beliebigen Stellen im Rest $R_a$ befinden; vorzugsweise ist $R_a$ mit einem oder zwei Substituenten versehen, die sich bevorzugt in der 1- und/oder 2- oder 3-Position des Restes $R_a$ befinden. Als Substituenten sind sauerstoff- und/oder halogenhaltige Reste geeignet. Besonders geeignet sind Hydroxy-, Acyloxy- und Chlorsubstituenten.

Als ungesättigte Reste kommen Alkenylgruppen mit bis zu zwei Doppelbindungen im Rest $R_a$ zur Anwendung. Besonders geeignet sind solche Alkenylgruppen, bei denen sich eine Doppelbindung in Position 1 befindet.

Bevorzugt als Gruppen $R_a$ sind die Methyl-, Ethyl-, Propyl-, Butyl-, Hexyl-, Decyl-, 2-Methylpropyl-, 3-Methylbutyl-, 2-Heptyl-, Vinyl-, Hydroxymethyl-, 2-Chlorethyl-, S1-Acetoxyethyl-, R1-Acetoxyethyl und 1,2-Diacetoxy-butylgruppe.

Die für $R_b$ stehenden Gruppen können eine Halogen-, Azido-, Amino-, Monoalkylamino-, Dialkylamino-, Monoacylamino-, Diacylamino-, Hydroxy-und Acyloxygruppe sein. Bevorzugt ist die Chlor-, Acetoxy-, Propionyloxy-, Butyryloxy-, Isobutyryloxy-, Hexanoyloxy-, Decanoyloxy-, Dimethylamino- und Acetylaminogruppe.

Die erfindungsgemäßen Verbindungen gemäß Formel I

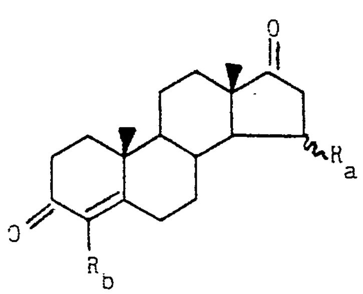

(I) ,

worin

$R_a$ einen gesättigten oder ungesättigten, gegebenenfalls substituierten Alkylrest mit 1-10 Kohlenstoffatome, wobei $R_a$ in der α- oder β-Position steht,

$R_b$ ein Halogen, eine Azido-, eine $-OR_1$ oder $-NR_2R_3$-Gruppe, wobei $R_1$ ein Wasserstoffatom oder eine Acylgruppe mit 1-10 Kohlenstoffatomen und $R_2$ und $R_3$ gleich oder verschieden sind und jeweils ein Wasserstoffatom, eine Alkyl-oder Acylgruppe mit jeweils 1-10 Kohlenstoffatomen darstellen, bedeuten, können hergestellt werden, indem man

a) eine Verbindung der allgemeinen Formel II

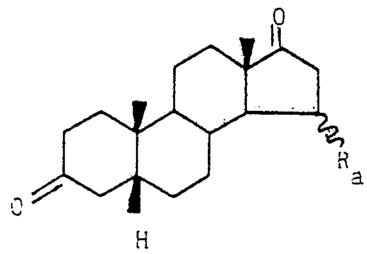

(II) .

worin

$R_a$ einen gesättigten oder ungesättigten, gegebenenfalls substituierten Alkylrest mit 1-10 Kohlenstoffatomen bedeutet, wobei $R_a$ in der α- oder β-Position steht,

mit Sauerstoff in Gegenwart von Basen oxidiert und das sigh gebildete 4-Enol gegebenenfalls verestert, oder

b) ein Epoxid der allgemeinen Formel III,

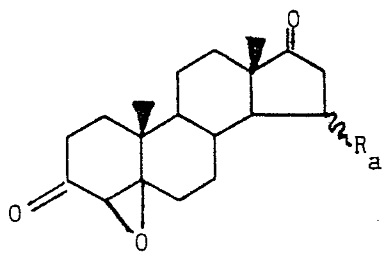

(III) ,

worin

$R_a$ einen gesättigten oder ungesättigten, gegebenenfalls substituierten Alkylrest mit 1-10 Kohlenstoffatomen bedeutet, wobei $R_a$ in der α- oder β-Position steht, unter protonierenden Bedingungen zur 4-Enolverbindung öffnet und gegebenenfalls verestert, oder mit einem Nucleophil, wie zum Beispiel Chlorid oder Azid, öffnet, dehydratisiert und gegebenenfalls die 4-Azido-Verbindung zur 4-Aminoverbindung hydriert und die Aminogruppe gegebenenfalls alkyliert oder acyliert.

Die Oxidation von Verbindungen der allgemeinen Formel II zu Verbindungen der allgemeinen Formel I verläuft mit Sauerstoff in Gegenwart eines Alkalialkoholates, wie Kalium-tert.-butylat, in einem Alkohol-Amin-Gemisch, wie tert.-Butanol-Triethylamin-Gemisch (7:3), bei Temperaturen zwischen 0 und 20°C, vorzugsweise bei 0-10°C. Das erhaltene 4-Enol wird gegebenenfalls mit einem Säureanhydrid in Gegenwart von Pyridin oder Triethylamin verestert. Für diese Veresterung eignen sich aber auch andere, dem Fachmann bekannte Methoden, siehe Tetrahedron 36, 2409 (1980). Die Verbindungen der allgemeinen Formel I können auch erhalten werden, indem die Epoxide der allgemeinen Formel III in Gegenwart eines Protonenspenders, wie Schwefelsäure in Eisessig oder dem sauren Ionenaustauscher Amberlyst 15, zum 4-Enol geöffnet werden. Der 4-Chlorsubstituent läßt sich einführen, indem Verbindungen der allgemeinen Formel III, in einer Eisessiglösung, die mit Chlorwasserstoffgas gesättigt ist, zur Reaktion gebracht werden. Unter diesen Reaktionsbedingungen tritt neben der nucleophilen Öffnung des Epoxids mit Chlorid eine Dehydratisierung ein. Das 4-Azidoderivat wird durch Reaktion des Epoxids der allgemeinen Formel III mit Natriumazid in Dimethylsulfoxid in Gegenwart von Schwefelsäure bei Temperaturen zwischen 20-150°C, vorzugsweise 60°C, erhalten. Die Reduktion der 4-Azido- in die 4-Aminoverbindung erfolgt mit Triphenylphosphin durch Rückflußsieden in einem Tetrahydrofuran-Wasser-Gemisch. Die Alkylierung und Acylierung der Aminoverbindung erfolgt nach dem den Fachmann bekannten Verfahren, beispielsweise nach der deutschen Offenlegungsschrift 3604179.

Die Verbindungen der allgemeinen Formel II

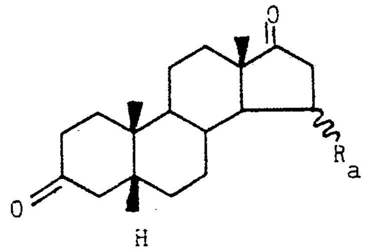

(II) ,

können ausgehend von 3β-Hydroxy-5,15-androstadien-17-on [J.Chem.Soc.(C)416, (1968)] wie folgt hergestellt werden:
Durch Michael-Addition von Cyanid erfolgt die Einführung einer β-Cyanogruppe in Position 15 am Steroidgerüst, anschließend wird das 17-Keton mit Natriumborhydrid zur 17β-Hydroxyverbindung reduziert. Die β-Cyanoverbindung läßt sich - wenn es gewünscht wird - mit Kalium-tert.-butylat in Tetrahydrofuran in die α-Cyanoverbindung umwandeln. Unabhängig davon, ob man sich in der α- oder β-Cyanoreihe befindet, lassen sich beide isomeren Cyanoverbindungen mit Diisobutylaluminiumhydrid in die entsprechenden 15-Aldehyde überführen. Die isomeren Aldehyde sind dann die Ausgangsverbindungen für die 15α- oder 15β-substituierten Androstendione.

Über die Sequenz Kettenverlängerung durch Wittig-Reaktion, metallkatalysierte Hydrierung der Doppelbindung, Oppenauer-Oxidation des Diols zum Dion und metallkatalysierte Hydrierung der 4-Doppelbindung unter basischen Bedingungen gelangt man zu Verbindungen der allgemeinen Formel II. Die 15-Methylverbindungen werden erhalten, indem die Aldehyde mittels Wolff-Kishner-Reaktion reduziert werden.

Darüber hinaus lassen sich die Verbindungen der allgemeinen Formel III,

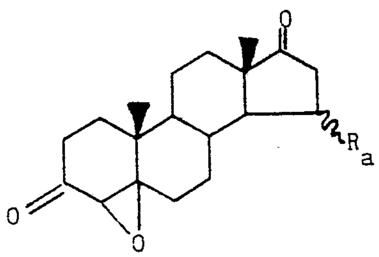

(III) ,

$R_a$ ist β-ständig, wie folgt herstellen:

Ausgehend von 3β-Hydroxy-5,15-androstadien-17-on wird der 15β-Substituent über eine kupferkatalysierte Grignardreaktion eingeführt. Durch anschließende Oxidation nach Oppenhauer erhält man ein 3,17-Dion, welches sich durch Epoxidierung (zum Beispiel mit Wasserstoffperoxid in methanolischer Kaliumhydroxidlösung) in Verbindungen der allgemeinen Formel III überführen läßt. Verbindungen der allgemeinen Formel III, mit α-ständigem $R_a$, können ausgehend vom bekannten 15α-Ethoxycarbonylmethyl-4-androsten-3,17-dion [Steroids 245(1982)] nach der folgenden Synthesesequenz unter Anwendung an sich bekannter Methoden hergestellt werden.

Das Diketon wird in den Positionen 3 und 17 mit Ethylenglykol ketalysiert, anschließend wird der Ester mit Lithiumaluminiumhydrid zum Alkohol reduziert, die Ketalschutzgruppen entfernt, die 15α-Hydroxyethyl- in die 15α-Chlorethylgruppe überführt [J.Am.Chem.Soc.88, 3440 (1966)] und anschließend unter den bereits beschriebenen Bedingungen die 4-Doppelbindung epoxidiert.

Ein Zugang zu Verbindungen der allgemeinen Formel III, bei denen der Rest $R_a$ einen sauerstoffhaltigen Substituenten, wie eine Hydroxyl- oder Acetoxygruppe darstellt, werden wie nachstehend beschrieben, hergestellt:

Ausgehend vom entsprechenden 15-Carbaldehyd verläuft die Synthese über eine Wittig- oder Corey-Reaktion, danach wird reduziert oder der nach der Wittig-Reaktion entstandene 15-Alkenylrest durch Epoxidierung mit m-Perbenzoesäure und anschließende reduktive Öffnung des Epoxids mit Lithiumaluminiumhydrid oder Methyllithium oder durch Umsetzung mit N-Methyl-Morpholin-N-Oxid in Gegenwart von Osmiumtetroxid hydroxyliert und gegebenenfalls acetyliert.

Die Wittig-Reaktion wird in Pure and Appl.Chem. 52,771 (1980) und die Corey-Reaktion in J.Am.Chem.Soc.84,3782(1962) und 87, 1353 (1965) beschrieben.

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimulierten Tumoren, wie

zum Beispiel Mammakarzinom oder Prostatahyperplasie.

Die erfindungsgemäßen Verbindungen sich auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulation oder Implantation durch Östrogenentzug zu hemmen.

Wahrscheinlich sind Aromatasehemmer auch zur Behandlung des drohenden Herzinfarktes geeignet, da erhöhte Östrogenspiegel beim Mann einem Herzinfarkt vorangehen können.

Bekannte, eine die Aromatase hemmende Wirkung aufweisende Steroidsubstanzen sind beispielsweise $\Delta^1$-Testolacton (US-PS Nr. 27 44 120), 4-Hydroxy-androst-4-en-3,17-dion und Ester davon [siehe zum Beispiel US-PS Nr. 42 35 983], 1-Methyl-1,4-androstadien-3,17-dion (DE-OS 33 22 285), 4-Amino-4,6-androstadien-3,17-dion (DE-OS 36 04 179) und 6-Methylen-1,4-androstadien-3,17-dion (DE-OS 36 22 841).

Im Vergleich zu dem bekannten Aromatasehemmern wird die Serum-Östradiolkonzentration mit den erfindungsgemäßen Verbindungen bei oraler Applikation stärker gesenkt. Der folgende tierexperimentelle Test zeigt die Wirksamkeit der erfindungsgemäßen Verbindungen an der Ratte.

Invantile weibliche Ratten reagieren auf PMSG (Pregnant Mare's Serum Gonadotropin)-Behandlung mit einheitlich erhöhter Steroidsynthese. Die Aromataseaktivität kann durch die Beeinflussung der PMSG-stimulierten Östrogenbildung gemessen werden. Im sogenannten PMSG-Test werden 21 Tage alte weibliche Ratten alle 2 Tage insgesamt 7 mal mit je 100 I.U. PMSG subkutan vorbehandelt. 1 Stunde vor und 8 Stunden nach der letzten PMSG-Applikation ($d_{12}$) erhalten die Tiere die Testsubstanz in 0,1 ml Benylbenzoat/Rizinusöl (1 + 9) durch subkutane Injektion. Die Kontrolltiere erhalten nur das Vehikel. 24 Stunden nach der letzten PMSG-Applikation werden die Tiere getötet. Im Serum wird Östradiol radioimmunologisch geprüft. Für jede Gruppe von 10 Tieren wird der Mittelwert der Östradiolkonzentration in nMol/l mit der Standardabweichung errechnet. Die Signifikanzen der Unterschiede zur Kontrollgruppe werden durch die Varianzanalyse geprüft.

Es wird die prozentuale Hemmung gegen die PMSG-Kontrolle berechnet. Der bekannte Aromatasehemmer 4-Hydroxy-4-androsten-3,17-dion (A) zeigt im geprüften Dosisbereich (2 x 10 mg) bei oraler Applikation keine Wirksamkeit. Die Verbindung 15β-Ethyl-4-hydroxy-4-androsten-3,17-dion (B) zeigt eine Hemmunng von 19 % und 4-Acetoxy-15β-ethyl-4-androsten-3,17-dion (C) eine von 33 %.

Tabelle

PMSG-Test bei infantilen Ratten, oral

| | Dosis mg/Tier 2 x p.o. | Konzentration im Serum Östradiol nMol/l | % Hemmung |
|---|---|---|---|
| PMSG-Kontrolle | - | 3,49 ± 0,23 | - |
| A | 10 | 3,45 ± 0,18 | 0 |
| B | 10 | 2,83 ± 0,29 | 19 |
| C | 10 | 2,34 ± 0,46 | 33 |

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,001 - 100 mg/kg Körpergewicht, vorzugsweise 0,01-20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, einen Suspendier- oder Emulgiermittels verwendet. Beispielsweise für verwendete Öle sind zum Beispiel Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone, wie zum Beispiel Silikonkautschuk. Die Wirkstoffe können außerdem zur perkutanen Applikation zum Beispiel in ein Pflaster eingearbeitet werden.

Die Erfindung betrifft somit auch pharmazeutische Präparate und die Verwendung der neuen Verbindungen der allgemeinen Formel I zur Herstellung dieser Präparate zur Behandlung von östrogen-bedingten Krankheiten.

**Beispiel 1**

**4-Hydroxy-15α-methyl-4-androsten-3,17-dion**

a) 50,3 g 3β-Hydroxy-5,15-androstadien-17-on [J.Chem.Soc., (C), 416 (1968)] werden in 1,06 l Tetrahydrofuran mit 60,8 g Kaliumcyanid in 350 ml Wasser versetzt und 15 Minuten unter Rückfluß gesiedet. Dann wird mit Wasser verdünnt und zweimal mit Essigester extrahiert. Man erhält 53,0 g 15β-Cyano-3β-hydroxy-5-androsten-17-on als Rohprodukt.

b) 22,8 g 15β-Cyano-3β-hydroxy-5-androsten-17-on werden in 600 ml Methanol und 300 ml Tetrahydrofuran mit 11,5 g Natriumborhydrid und 46 ml Wasser portionsweise versetzt und 15 Minuten bei 20° C gerührt. Dann wird in 10 Liter Eiswasser gegossen, der Niederschlag abgesaugt, mit Wasser neutral gewaschen und im Trockenschrank bei 70°C über Nacht getrocknet. Das Rohprodukt wird aus Aceton/Toluol (1:1) kristallisiert. Man erhält 20,6 g 15β-Cyano-5-androsten-3β,17β-diol vom Schmelzpunkt 251-255° C.

c) 18,6 g 15β-Cyano-5-androsten-3β,17β-diol werden in 700 ml Tetrahydrofuran unter Argon mit 36 g Kalium-tert.-butylat in 300 ml Tetrahydrofuran 15 Minuten am Rückfluß gekocht. Beim Abkühlen kristallisiert das Reaktionsprodukt aus. Durch Absaugen der Kristalle werden 16,4 g 15α-Cyano-5-androsten-3β,17β-diol erhalten.

d) 5 g 15α-Cyano-5-androsten-3β,17β-diol werden in 200 ml Tetrahydrofuran bei -15°C mit 150 ml Diisobutylaluminiumhydridlösung (1.0 M in Tetrahydrofuran) tropfenweise versetzt und 2 Stunden bei 0° C nachgerührt. Dann werden 300 ml 10% wäßrige Weinsäure portionsweise zugegeben, in 400 ml Wasser und 200 g Eis gefällt, abgesaugt, mit Wasser gewaschen und getrocknet. Nach der Kristallisation aus Aceton erhält man 4,0 g 3β,17β-Dihydroxy-5-androsten-15β-carbaldehyd vom Schmelzpunkt 171-173° C (Zersetzung).

e) 4,4 g 3β,17β-Dihydroxy-5-androsten-15α-carbaldehyd werden in 25 ml Diethylenglykol mit 5,3 ml Hydrazinhydroxid 1,5 Stunden bei 130°C unter Argon gerührt. Dann wird 4,4 g Kaliumhydroxidpulver zugegeben und 1,5 Stunden bei 225°C am Wasserabscheider erhitzt. Nach dem Abkühlen wird in salzsaures Eiswasser gegossen, abgesaugt, getrocknet, in Methanol aufgenommen, filtriert, das Filtrat eingeengt und aus Aceton kristallisiert. Man erhält 2,3 g 15 α-Methyl-5-androsten-3β,17β-diol vom Schmelzpunkt 178-182° C.

f) 1,8 g 15α-Methyl-5-androsten-3β,17β-diol werden in 100 ml Toluol und 500 ml Cyclohexanon am Wasserabscheider portionsweise mit 5 g Aluminiumtriisopropylat über 4 Stunden versetzt. Nach dem Abkühlen wird angesäuert, mit Wasser verdünnt und mit Dichlormethan extrahiert. Nach Chromatographie an Kieselgel erhält man zu 1,29 g 15α-Methyl-4-androsten-3,17-dion vom Schmelzpunkt 129-131° C umgesetzt.

g) 1,08 g 15α-Methyl-4-androsten-3,17-dion in 18 ml Methanol werden mit 1,0 g Kaliumhydroxidpulver und 0,1g 10% Palladium auf Aktivkohle versetzt und 9 Stunden bei 20°C unter Wasserstoff geschüttelt. Dann wird mit Eisessig versetzt, der Katalysator abfiltriert, mit Essigester verdünnt, mit Wasser gewaschen, über Natriumsulfat getrocknet und eingeengt. Man erhält 1,1 g rohes 15α-Methyl-5β-androstan-3,17-dion.

h) 310 mg rohes 15α-Methyl-5β-androstan-3,17-dion in 7 ml tert.-Butanol und 3 ml Triethylamin werden mit 1890 mg Kalium-tert.-butylat versetzt und 2 Stunden bei 3° C mit Sauerstoffgas behandelt. Dann wird mit Eisessig versetzt, eingeengt, in Essigester aufgenommen, dreimal mit Wasser gewaschen, über Natriumsulfat getrocknet und nach Chromatographie erhält man 111 mg 4-Hydroxy-15α-methyl-4-androsten-3,17-dion vom Schmelzpunkt 150-152° C.

**Beispiel 2**

**15β-Ethyl-4-hydroxy-4-androsten-3,17-dion**

a) 12 g Magnesium-Späne werden in 100 ml Tetrahydrofuran suspendiert und mit 38 ml Ethylbromid in 500 ml Tetrahydrofuran so versetzt, daß die Temperatur 45°C nicht überschreitet. Nach beendeter Zugabe wird auf 0° C gekühlt und mit 6,6 g Kupfer-(I)-jodid 15 Minuten nachgerührt. Dann gibt man eine Lösung von 20 g 3β-Hydroxy-5,15-androstadien-17-on [R.W. Kelly and P.J. Sykes, J.Chem.Soc., (C), 416(1968)] in 300 ml Tetrahydrofuran tropfenweise hinzu und rührt 15 Minuten bei 0°C nach. Anschließend wird das überschüssige Grignardreagenz mit wäßriger Ammoniumchloridlösung zersetzt. Man extrahiert mit Essigester und wäscht mit Wasser neutral. Nach dem Einengen erhält man 22,3 g Rohprodukt, daß an Kieselgel mit Hexan/Aceton chromatographiert wird. Es werden 15,9 g 15β-Ethyl-3β-hydroxy-5-androsten-17-on vom Schmelzpunkt 108-109° C erhalten.

b) 13,3 g 15β-Ethyl-3β-hydroxy-5-androsten-17-on werden in 680 ml Toluol und 200 ml Cyclohexanon gelöst und am Wasserabscheider portionsweise mit 21 g Aluminiumtriisopropylat innerhalb von 1,5 Stunden versetzt. Nach dem Abkühlen wird mit verdünnter Schwefelsäure angesäuert, die Wasserphase abgetrennt und mit Dichlormethan extrahiert. Die vereinigten organischen Phasen werden einer Wasserdampfdestillation unterworfen und in Dichlormethan aufgenommen, über Natriumsulfat getrocknet und eingeengt. Nach Chromatographie an Kieselgel mit Hexan/Essigestger werden 7,3 g 15β-Ethyl-4-androsten-3,17-dion als Schaum mit $[\alpha]_D$: +112,50 ($CHCl_3$) erhalten.

c) 4,1 g 15β-Ethyl-4-androsten-3,17-dion werden in 125 ml Methanol gelöst, bei 0°C mit 16 ml 30% Wasserstoffperoxid versetzt und 15 Minuten nachgerührt. Dann wird eine Lösung von 1,44 g Kaliumhydroxid in 8,6 ml Wasser hinzugefügt und 15 Stunden nachgerührt. Anschließend wird eine

Natriumdihydrogenphosphatlösung zugesetzt, die Wasserphase zweimal mit Ethylacetat extrahiert, die vereinigten organischen Phasen mit Natriumchloridlösung gewaschen, über Natriumsulfat getrocknet und im Vakuum eingeengt. Nach Chromatographie erhält man 2,63 g 15β-Ethyl-4,5-epoxy-androstan-3,17-dion mit einem Schmelzpunkt von 191-193° C.

d) 2,6 g 15β-Ethyl-4,5-epoxy-androstan-3,17-dion werden in 50 ml Dioxan mit 2,6 g saurem Ionenaustauscher (Amberlyst 15) 10 Stunden bei 50° C gerührt. Nach dem Abfiltrieren des Katalysators und Chromatographie erhält man 1,19 g 15β-Ethyl-4-hydroxy-4-androsten-3,17-dion vom Schmelzpunkt 168-169° C.

$[\alpha]_D$: +121,4° ($CHCl_3$), UV: $+\xi_{277}$ 12670.

**Beispiel 3**

**4-Acetoxy-15β-ethyl-4-androsten-3,17-dion**

671 mg 15β-Ethyl-4-hydroxy-4-androsten-3,17-dion werden in 7 ml Pyridin bei 0° C mit 3,5 ml Acetanhydrid 15 Stunden gerührt. Dann wird mit schwefelsaurem Eiswasser gefällt, mit Dichlormethan extrahiert, mit Natriumhydrogencarbonat-Natriumsulfat getrocknet und im Vakuum eingeengt. Nach der Chromatographie erhält man 464 mg 4-Acetoxy-15β-ethyl-4-androsten-3,17-dion vom Schmelzpunkt 173-175° C.

$[\alpha]_D$: +126,6° ($CHCl_3$), UV: $\xi_{245}$ 15200, $\xi_{205}$ 2670

**Beispiel 4**

**15α-Ethyl-4-hydroxy-4-androsten-3,17-dion**

a) 50,3 g 3β-Hydroxy-5,15-androstadien-17-on [R.W. Kelly and P.J. Sykes, J.Chem.Soc.,(C), 416(1968)] werden in 1,06 l Tetrahydrofuran mit 60,8 g Kaliumcyanid in 350 ml Wasser versetzt und 15 Minuten am Rückfluß gekocht. Dann wird mit Wasser verdünnt und zweimal mit Essigester extrahiert. Man erhält 53 g 15β-Cyano-3β-hydroxy-5-androsten-17-on als Rohprodukt.

b) 50 g rohes 15β-Cyano-3β-hydroxy-5-androsten-17-on werden in 950 ml Tetrahydrofuran bei -20° C mit 950 ml Diisobutylaluminiumhydrid-Lösung (1,2 M in Toluol) 2,5 Stunden gerührt. Dann wird mit verdünnter Schwefelsäure versetzt, über Kieselgur abgesaugt, die organische Phase mit Natriumhydrogencarbonatlösung neutral gewaschen, über Natriumsulfat getrocknet und im Vakuum eingeengt. Es werden 50,1 g roher 3β,17β-Dihydroxy-5-androsten-15-carbaldehyd erhalten.

c) 50,1 g roher 3β,17β-Dihydroxy-5-androsten-15-carbaldehyd werden in 500 ml Pyridin bei 20° C mit 250 ml Acetanhydrid 3,5 Stunden gerührt und wie unter Beispiel 3 beschrieben aufgearbeitet. Man erhält 52,0 g 3β,17β-Diacetoxy-5-androsten-15-carbaldehyd.

d) 52,0 g 3β,17β-Diacetoxy-5-androstan-15-carbaldehyd werden 30 Minuten bei 20° C in 500 ml mit Salzsäuregas gesättigtem Eisessig gerührt. Dann wird in Eiswasser gefällt, in Dichlormethan aufgenommen, mit Natriumhydrogencarbonatlösung gewaschen, über Natriumsulfat getrocknet und im Vakuum eingeengt. Nach Chromatographie erhält man 26,7 g 3β,17β-Diacetoxy-5-androsten-15α-carbaldehyd vom Schmelzpunkt 134-135° C.

$[\alpha]_D$: -51° ($CHCl_3$).

e) 19,7 g 3β,17β-Diacetoxy-5-androsten-15α-carbaldehyd werden bei 20° C zu einer Mischung von 56 g Triphenylmethylphosphoniumbromid in 490 ml Dimethylsulfoxid und 17,6 g Kalium-tert.-butylat gegeben und 2 Stunden bei 20° C gerührt. Dann wird mit verdünnter Schwefelsäure gefällt und zweimal mit Essigester extrahiert, die vereinigte organische Phase wird mit Wasser und Natriumchloridlösung gewaschen, über Natriumsulfat getrocknet und im Vakuum eingeengt. Das erhaltene Produkt wird in 500 ml 0,2 n methanolischer Kaliumhydroxid-Lösung 3,5 Stunden bei 20° C gerührt. Danach wird mit Essigsäure neutralisiert, mit Eiswasser gefällt, mit Wasser gewaschen und im Vakuum bei 70° C eingeengt. Man erhält 32 g rohes 15α-Vinyl-5-androsten-3β,17β-diol, das mit Triphenylphosphin verunreinigt ist.

f) 32 g rohes 15α-Vinyl-5-androsten-3β,17β-diol werden in 450 ml Methanol mit 3,2 g Palladium (10 %) auf Aktivkohle 1,5 Stunden unter Wasserstoff geschüttelt. Der Katalysator wird über Kieselgur abfiltriert, mit Dichlormethan nachgewaschen und die organische Phase im Vakuum eingeengt. Man erhält 32 g rohes 15α-Ethyl-5-androsten-3β,17β-diol.

g) 32 g rohes 15α-Ethyl-5-androsten-3β,17β-diol werden in 1,5 Liter Toluol und 483 ml Cyclohexanon am Wasserabscheider portionsweise mit 72 g Aluminiumtriisopropylat über 4 Stunden versetzt. Die Aufarbeitung erfolgt analog Beispiel 2 b). Man erhält 52 g Rohprodukt, daß nach Chromatographie 11,3 g 15α-Ethyl-4-androsten-3,17-dion vom Schmelzpunkt 158-159° C ergibt.

$[\alpha]_D$: +209,7° ($CHCl_3$)

h) 11,3 g 15α-Ethyl-4-androsten-3,17-dion werden in 340 ml Methanol bei 0° C mit 45,2 ml 30%igem Wasserstoffperoxid 15 Minuten gerührt und anschließend mit einer Lösung von 4 g Kaliumhydroxid in 25 ml Wasser versetzt. Aufarbeitung erfolgt analog Beispiel 2 c). Es werden 11,3 g Rohprodukt erhalten. Nach chromatographischer Reinigung beträgt die Ausbeute 7,9 g 4,5-Epoxy-15α-ethyl-androstan-3,17-dion vom Schmelzpunkt 183-184° C.

i) 2,5 g 4,5-Epoxy-15α-ethyl-androstan-3,17-dion werden analog Beispiel 2 d) zu 1,28 g 15α-Ethyl-4-hydroxy-4-androsten-3,17-dion vom Schmelzpunkt 165-166° C umgesetzt.

$[\alpha]_D$: +196,1°.

**Beispiel 5**

**4-Acetoxy-15α-ethyl-4-androsten-3,17-dion**

2,5 g rohes 15α-Ethyl-4-hydroxy-4-androsten-3,17-dion werden in 20 ml Pyridin bei 20°C mit 10 ml Acetanyhdrid 2 Stunden gerührt. Die Aufarbeitung erfolgt analog Beispiel 3 . Man erhält 1,27 g 4-Acetoxy-15α-ethyl-4-androsten-3,17-dion vom Schmelzpunkt 226-227°C. $[\alpha]_D$: 193.8° ($CHCl_3$). $\xi_{246}$ 15100.

**Beispiel 6**

**4-Hydroxy-15α-hydroxymethyl-4-androsten-3,17-dion**

a) 2,8 g 3β,17β-Dihydroxy-5-androsten-15-carbaldehyd werden in 150 ml Tetrahydrofuran mit 4,2 g Lithiumaluminiumhydrid 1,2 Stunden bei 0° C unter Argon gerührt. Anschließend wird mit schwefelsaurem Eiswasser zersetzt, abgesaugt, mit Wasser gewaschen, getrocknet, mit Chloroform/Methanol aufgenommen, eingeengt und kristallisiert. Man erhält 2,3 g 15α-Hydroxymethyl-5-androsten-3β,17β-diol vom Schmelzpunkt 250-251° C.

b) 2,3 g 15α-Hydroxymethyl-5-androsten-3β,17β-diol werden in 60 ml Dimethylformamid mit 0,4 g Bleidiacetat und 5,5 ml Acetanhydrid 10,5 Stunden bei 20°C gerührt. Es wird mit Eiswasser gefällt, mit Wasser gewaschen, in Chloroform/Methanol aufgenommen und aus Aceton kristallisiert. Man erhält 1,7 g 15α-Acetoxymethyl-5-androsten-3β,17β-diol vom Schmelzpunkt 165-167° C.

c) 1,5 g 15α-Acetoxymethyl-5-androsten-3β,17β-diol werden analog Beispiel 4 g) zu 1,1 g 15α-Acetoxymethyl-4-androsten-3,17-dion vom Schmelzpunkt 127-129° C umgesetzt.

d) 0,7 g 15α-Acetoxymethyl-4-androsten-3,17-dion werden 1,7 Stunden bei 20°C mit 14 ml einer 0,1%igen Kaliumhydroxidlösung in Methanol gerührt. Anschließend wird mit Eisessig neutralisiert, mit Dichlormethan verdünnt, mit Wasser gewaschen, über Natriumsulfat getrocknet und eingeengt. Man erhält 700 mg 15α-Hydroxymethyl-4-androsten-3,17-dion vom Schmelzpunkt 173-174° C.

e) 475 mg 15α-Hydroxymethyl-4-androsten-3,17-dion werden analog Beispiel 1 f) hydriert. Man erhält 490 mg 15α-Hydroxymethyl-5β-3,17-dion (roh).

f) 600 mg 15α-Hydroxymethyl-5β-androstan-3,10 dion werden in 5 ml Toluol mit 0,6 ml Dihydropyran und 6 mg p-Toluolsulfonsäure 1 Stunde bei 20°C gerührt. Es wird Pyridin zugesetzt, mit Essigester verdünnt, mit Wasser gewaschen, über Natriumsulfat getrocknet und eingeengt. Man erhält 860 mg rohes 15α-(Tetrahydropyran-2-yloxy-methyl)-5β-androstan-3,17-dion.

g) 620 mg 15α-(Tetrahydropyran-2-yloxy-methyl)-5β-androstan-3,17-dion werden analog Beispiel 1 g) zu 630 mg 4-Hydroxy-15α-(tetrahydropyran-2-yloxy-methyl)-4-androsten-3,17-dion umgesetzt.

h) 280 mg 4-Hydroxy-15α-(tetrahydropyran-2-yloxy-methyl)-4-androsten-3,17-dion werden in 5 ml Methanol mit 1,5 ml 10%iger wäßriger Oxalsäure 30 ml am Rückfluß erhitzt. Es wird in Eiswasser gegossen, abgesaugt, in Dichlormethan gelöst, mit Wasser gewaschen, über Natriumsulfat getrocknet und eingedampft. Nach Chromatographie und Kristallisation aus Aceton/Hexan (1:1) erhält man 133 mg 4-Hydroxy-15α-hydroxymethyl-4-androsten-3,17-dion vom Schmelzpunkt 189-190° C.

**Beispiel 7**

**15α-(2-Chlorethyl)-4-acetoxy-4-androsten-3,17-dion**

a) 25 g 15α-Ethoxycarbonylmethyl-4-androsten-3,17-dion (Y.Miyake, Y.Kubo, S.Iwabuchi and M.Kojina, Steroids(1982),245) in 750 ml Toluol werden mit 50 ml Ethylenglykol und 500 mg p-Toluolsulfonsäure 7 Stunden am Wasserabscheider erhitzt. Es wird abgekühlt, mit Essigester verdünnt, mit Natriumhydrogencarbonat- und Kochsalzlösung gewaschen und im Vakuum eingeengt. Man erhält 39,3 g rohe 15α-Ethoxycarbonylmethyl-3,3;17,17-bis(ethylendioxy)-5-androsten.

b) 39,3 g rohes 15α-Ethoxycarbonylmethyl-3,3;17,17-bis(ethylendioxy)-5-androsten in 800 ml Tetrahydrofuran werden bei 0°C mit 3,93 g Lithiumaluminiumhydrid portionsweise versetzt und 2 Stunden gerührt. Dann werden 4 ml Wasser, 15% Natronlauge und 12 ml Wasser zugetropft, 1 Stunde nachgerührt, über Celite 163 abgesaugt, mit Dichlormethan nachgewaschen und eingeengt. Man erhält 29,7 g rohes 3,3;17,17-Bis-(ethylendioxy)-15α-(hydroxyethyl)-5-androsten.

c) 29,7 g rohes 3,3;17,17-Bis(ethylendioxy)-15α-(2-hydroxyethyl)-5-androsten in 600 ml Aceton werden mit 120 ml 1 n Schwefelsäure 2 Stunden am Rückfluß gekocht. Dann wird mit Essigester verdünnt, mit Kochsalzlösung gewaschen und eingeengt. Man erhält 22,3 g rohes 15α-(2-Hydroxyethyl)-4-androsten-3,17-dion.

d) 10,3 g 15α-(2-Hydroxyethyl)-4-androsten-3,17-dion werden in 100 ml Dichlormethan, 100 ml Tetrachlorkohlenstoff und 40 ml Pyridin gelöst und mit 10 g Triphenylphosphin 18 Stunden bei 20°C gerührt. Dann wird mit Essigester verdünnt, mit 1 n Salzsäure und Kochsalzlösung gewaschen und eingeengt. Nach Chromatographieren erhält man 11,1 g 15α-(2-Chlorethyl)4-androsten-3,17-dion vom Schmelzpunkt 162-164° C.

e) 4,3 g 15α-(2-Chlorethyl)-4-androsten-3,17-dion werden analog Beispiel 2c) und Beispiel 2 d) zu 1,2 g

rohem 15α-(2-Chlorethyl)-4-hydroxy-4-androsten-3,17-dion umgesetzt.

f) 1,2 g rohes 15α-(2-Chlorethyl)-4-hydroxy-4-androsten-3,17-dion werden analog Beispiel 3 zu 230 mg 15α-(2-Chlorethyl)-4-acetoxy-4-androsten3,17-dion vom Schmelzpunkt 187-191° C umgesetzt.

**Beispiel 8**

**S15α-(1-Acetoxyethyl)-4-hydroxy-4-androsten-3,17-dion**

a) 22 g 3β,17β-Diacetoxy-5-androsten-15α-carbaldehyd werden in 350 ml Dimethylformamid bei 210° C mit 30 g Trimethylsulfoniumjodid und 21 g Kalium-tert.-butylat 30 Minuten gerührt. Es wird in Eiswasser/Kochsalz eingefällt, filtriert, mit Wasser gewaschen, in Dichlormethan aufgenommen, mit Wasser gewaschen, über Natriumsulfat getrocknet und im Vakuum eingeengt. Man erhält 17,4 g Rohprodukt. Nach chromatographischer Reinigung erhält man 9,0 g 15α-(1,2-Epoxyethyl)-5-androsten-3β,17β-diol.

$[\alpha]_D$ -20,3°.

b) 5,5 g 15α-(1,2-Epoxyethyl)-5-androsten-3β,17β-diol in 105 ml Toluol und 21 ml Dihydropyran werden mit 105 mg p-Toluolsulfonsäure versetzt und 3 Stunden bei Raumtemperatur gerührt. Dann wird mit Essigester verdünnt, mit Natriumhydrogencarbonatlösung und Natriumchloridlösung gewaschen, über Natriumsulfat getrocknet und im Vakuum eingeengt. Nach der chromatographischen Reinigung erhält man 6,2 g 15α-(1,2-Epoxyethyl)-3β,17β-bis(tetrahydropyran-2-yloxy)-5-androsten.

$[\alpha]_D$ -5,6° (Pyridin).

c) 6,1 g 15α-(1,2-Epoxyethyl)-3β,17β-bis(tetrahydropyran-2-yloxy)-5-androsten in 122 ml Tetrahydrofuran werden bei 0° C langsam mit 1,83 g Lithiumaluminiumhydrid versetzt und 1,5 Stunden bei 0° C nachgerührt. Danach wird Essigester unter Kühlung zugetropft, 20 ml Wasser zugegeben, über Celite filtriert, mit Essigester nachgewaschen und im Vakuum eingeengt. Man erhält 6,2 g rohes S15α-(1-Hydroxyethyl)-3β,17β-bis(tetrahydropyran-2-yloxy)-5- androsten.

d) 6,2 g rohes S15α-(1-Hydroxyethyl)-3β,17β-bis(tetrahydropyran-2-yloxy)-5-androsten in 62 ml Pyridin und 31 ml Acetanhydrid werden 16 Stunden bei 20° C gerührt. Dann wird mit Eiswasser/Kochsalz gefällt, abfiltriert, mit Wasser gewaschen, in Dichlormethan aufgenommen, mit Wasser gewaschen, über Natriumsulfat getrocknet und im Vakuum zur Trockne eingeengt. Man erhält 6,7 g rohes S15α-(1-Acetoxyethyl)-3β,17β-bis(tetrahydropyran-2-yloxy)-5-androsten.

e) 6,7 g rohes S15α-(1-Acetoxyethyl)-3β,17β-bis(tetrahydropyran-2-yloxy)-5-androsten in 135 ml Methanol werden mit 13,4 ml Wasser und 6,7 g Oxalsäure versetzt und 15 Minuten am Rückfluß gekocht. Danach wird analog Beispiel 8 d) aufgearbeitet. Man erhält 4,7 g rohes S15α-(1-Acetoxyethyl)-5-androsten-3β,17β-diol.

f) 4,7 g rohes S15α-(1-Acetoxyethyl)-5-androsten-3β,17β-diol in 220 ml Toluol und 71,1 ml Cyclohexanon werden am Wasserabscheider gekocht. Innerhalb von 2 Stunden werden 7,8 g Aluminiumtriiospropylat portionsweise zugegeben. Danach wird das Rohprodukt analog Beispiel 2 b) aufgearbeitet und anschließend mit 450 ml Dichlormethan, 5,78 g Pyridiniumchlorochromat und 732 mg Natriumacetat versetzt und 5 Stunden bei 20° C gerührt. Nach Abtrennung der Feststoffe wird eingeengt und chromatographiert. Es werden 3,2 g S15α-(1-Acetoxyethyl)-4-androsten-3,17-dion vom Schmelzpunkt 197-198° C erhalten.

g) 3,1 g S15α-(1-Acetoxyethyl)-4-androsten-3,17-dion werden analog Beispiel 2 c) zu 2,79 g S15α-(1-Acetoxyethyl-4,5-epoxy-androstan-3,17-dion umgesetzt.

$[\alpha]_D$: +52,5° (Pyridin).

h) 2,7 g S15α-(1-Acetoxyethyl)-4,5-epoxy-androstan-3,17-dion werden analog Beispiel 2 d) zu 490 mg S15α-(1-Acetoxyethyl)-4-hydroxy-4-androsten-3,17-dion vom Schmelzpunkt 202-203° C umgesetzt.

$[\alpha]_D$ : +154,6° (CHCl3).

**Beispiel 9**

**R15α-(1-Acetoxyethyl)-4-hydroxy-4-androsten-3,17-dion**

a) 5,0 g 3β,17β-Dihydroxy-5-androsten-15-carbaldehyd werden in 50 ml Toluol mit 10 ml Dihydropyran sowie 50 mg Toluolsulfonsäure 45 Minuten bei 20° C gerührt. Es werden 5 ml Pyridin zugesetzt, mit Essigester verdünnt, mit Natriumhydrogencarbonatlösung gewaschen, über Natriumsulfat getrocknet und im Vakuum zur Trockne eingeengt. Man erhält nach Chromatographie 4,7 g 3β,17β-Bis(tetrahydropyran-2-yloxy)-5-androsten-15β-carbaldehyd.

$[\alpha]_D$ : -59,0° (Pyridin).

b) 76 g 3β,17β-Bis(tetrahydropyran-2-yloxy)-5-androsten-15β-carbaldehyd in 1 Liter Tetrahydrofuran werden bei 0° C mit 316 ml Methyllithium (1,6 m in Ether) langsam versetzt und 15 Minuten nachgerührt. Dann wird unter Kühlung mit Wasser versetzt, mit Essigester verdünnt, die organische Phase mit Wasser neutral gewaschen, über Natriumsulfat getrocknet und im Vakuum eingeengt. Man erhält 80,6 g rohes R15α-(1-Hydroxyethyl)-3β,17β-bis(tetrahydropyran-2-yloxy)-5-androsten.

c) 80,6 g rohes R15α-(1-Hydroxyethyl)-3β,17β-bis(tetrahydropyran-2-yloxy)-5-androsten werden analog Beispiel 8 d) zu 84,4 g rohem R15α-(1-Acetoxyethyl)-3β,17β-bis(tetrahydropyran-2-yloxy)-5-androsten umgesetzt.

d) 84,4 g rohes R15α-(1-Acetoxyethyl)-3β,17β-bis(tetrahydropyran-2-yloxy)-5-androsten werden analog Beispiel 8 e) zu 49,1 g rohem R15α-(1-Acetoxyethyl)-5-androsten-3β,17β-diol umgesetzt.

e) 29,9 g rohes R15α-(1-Acetoxyethyl)-5-androsten-3β,17β-diol werden analog Beispiel 4 g) umgesetzt. Nach Chromatographie erhält man 15,6 g R15α-(1-Acetoxyethyl)-4-androsten-3,17-dion.
$[\alpha]_D$ : +95,3°.

f) 15,4 g R15α(1-Acetoxyethyl)-4-androsten-3,17-dion werden analog Beispiel 2 c) zu 8,8 g R15α-(1-Acetoxyethyl)-4,5-epoxy-androstan-3,17-dion umgesetzt.

g) 4,9 g R15α-(1-Acetoxyethyl)-4,5-epoxy-androstan-3,17-dion werden analog Beispiel 2 d) zu 1,81 g R15α-(1-Acetoxyethyl)-4-hydroxy-4-androsten-3,17-dion vom Schmelzpunkt 136-145° C umgesetzt.
$[\alpha]_D$: +75,9°
UV$\varepsilon_{276}$: 11760

**Beispiel 10**

Durch Umsetzung von 3β,17β-Dihydroxy-5-androsten-15-α-carbaldehyd mit geeigneten Phosphoryliden analog Beispiel 4 e) und Weiterumsetzung analog Beispiel 4 f) - i) gelangt man zu:

a) 4- Hydroxy-15α-propyl-4-androsten-3,17-dion
Schmelzpunkt: 154-156° C.

b) 15α-Butyl-4-hydroxy-4-androsten-3,17-dion
Schmelzpunkt: 160-162° C.

c) 15α-Hexyl-4-hydroxy-4-androsten-3,17-dion
Schmelzpunkt: 93-95° C.

d) 15α-Decyl-4-hydroxy-4-androsten-3,17-dion
(Schaum)

e) 4-Hydroxy-15α-(2-methylpropyl)-4-androsten-3,17-dion.
Schmelzpunkt: 142-146° C.

f) 4-Hydroxy-15α-(3-methylbutyl)-4-androsten-3,17-dion
Schmelzpunkt: 135-137° C.

g) 15α-(hept-2-yl)-4-hydroxy-4-androsten-3,17-dion.
Schmelzpunkt: 111-113° C.

**Beispiel 11**

Durch Umsetzung der 4-Hydroxyverbindungen aus Beispiel 10 analog Beispiel 5 erhält man:

a) 4-Acetoxy-15α-propyl-4-androsten-3,17-dion
Schmelzpunkt: 191-192° C.

b) 4-Acetoxy-15α-butyl-4-androsten-3,17-dion
Schmelzpunkt: 169-171° C.

c) 4-Acetoxy-15α-hexyl-4-androsten-3,17-dion
Schmelzpunkt: 101-102° C.

d) 4-Acetoxy-15α-decyl-4-androsten-3,17-dion
(Schaum)

e) 4-Acetoxy-15α-(2-methylpropyl)-4-androsten-3,17-dion
Schmelzpunkt: 147-150° C

f) 4-Acetoxy-15α-(3-methylbutyl)-4-androsten-3,17-dion
Schmelzpunkt: 152-154° C

g) 4-Acetoxy-15α-(hept-2-yl)-4-androsten-3,17-dion
Schmelzpunkt: 120-122° C

**Beispiel 12**

**4-Chlor-15α-ethyl-4-androsten-3,17-dion**

Eine Lösung von 1,2 g 4,5-Epoxy-15α-ethyl-androstan-3,17-dion in 10 ml Eisessig wird mit 5 ml gesättigter Chlorwasserstoff-/Eisessiglösung 30 Minuten bei 20° C gerührt. Nach der Fällung in Eiswasser/Kochsalz und Chromatographie erhält man 850 mg 4-Chlor-15α-ethyl-4-androsten-3,17-dion vom Schmelzpunkt 147-149° C.

**Beispiel 13**

**4-Azido-15α-ethyl-4-androsten-3,17-dion**

Eine Lösung von 1,5 g 4,5-Epoxy-15α-ethyl-androstan-3,17-dion in 20 ml Dimethylsulfoxid wird mit 0,5 ml Schwefelsäure und 4,1 g Natriumazid 2 Stunden bei 60° C gerührt. Danach wird in salzsaures Eiswasser gefällt. Nach Absaugen und Chromatographie erhält man 0,88 g 4-Azido-15α-ethyl-4-androsten-3,17-dion vom Schmelzpunkt 148-150° C.

**Beispiel 14**

**4-Amino-15α-ethyl-4-androsten-3,17-dion**

800 mg 4-Azido-15α-ethyl-4-androsten-3,17-dion in 10 ml Tetrahydrofuran und 5 ml Wasser werden mit 1 g Triphenylphosphin 3 Stunden am Rückfluß erhitzt. Dann wird mit Essigester verdünnt, mit Wasser gewaschen, über Natriumsulfat getrocknet, vom Lösemittel befreit und chromatographiert. Man erhält 633 mg 4-Amino-15α-ethyl-4-androsten-3,17-dion vom Schmelzpunkt 152-154° C.

**Beispiel 15**

**15α-Ethyl-4-N-methylamino-4-androsten-3,17-dion**

1,7 g 4-Amino-15α-ethyl-4-androsten-3,17-dion in 40 ml Benzol werden mit 0,81 g Benzaldehyd 1,5 Stunden am Wasserabscheider Rückfluß gesiedet. Dann läßt man auf 20° C abkühlen und gibt 0,95 g Dimethylsulfat in 5 ml Benzol zu und siedet anschließend 30 Minuten. Danach wird mit Wasser versetzt, 0,5 Stunden bei 80° C gerührt, abgekühlt, die Wasserphase abgetrennt, zweimal mit Ether extrahiert, mit Natriumhydroxidlösung alkalisch gestellt, mit Essigester extrahiert, über Natriumsulfat getrocknet und im Vakuum eingeengt. Nach Chromatographie erhält man 0,97 g 15α-Ethyl-4-N-methylamino-4-androsten-3,17-dion vom Schmelzpunkt 114-117° C.

**Beispiel 16**

**4-Acetylamino-15α-ethyl-4-androsten-3,17-dion**

1,5 g 4-Amino-15α-ethyl-4-androsten-3,17-dion in 5 ml Pyridin werden bei 20° C mit 3 ml Acetanhydrid 2 Stunden gerührt. Dann wird unter Eiskühlung mit Wasser verdünnt, mit Essigester extrahiert, mit gesättigter Kochsalzlösung gewaschen, über Natriumsulfat getrocknet und eingeengt. Nach der chromatographischen Reinigung erhält man 1,4 g 4-Acetylamino-15α-ethyl-4-androsten-3,17-dion vom Schmelzpunkt 229-231° C.

**Beispiel 17**

Durch Anwendung geeigneter Säureanyhdride erhält man aus 15α-Ethyl-4-hydroxy-4-androsten-3,17-dion analog Beispiel 5 a):

a) 15α-Ethyl-4-propionyloxy-4-androstan-3,17-dion
Schmelzpunkt: 195-197° C.

b) 4-Butyryloxy-15α-ethyl-4-androsten-3,17-dion
Schmelzpunkt: 181-183° C.

c) 15α-Ethyl-4-isobutyryloxy-4-androsten-3,17-dion
Schmelzpunkt: 185-186° C

d) 15α-Ethyl-4-hexanoyloxy-4-androsten-3,17-dion
Schmelzpunkt: 153-155° C

c) 4-Decanoyloxy-15α-ethyl-4-androsten-3,17-dion
Schaum

**Beispiel 18**

**4-Hydroxy-15α-vinyl-4-androsten-3,17-dion**

a) 1,6 g 15α-Vinyl-5-androsten-3β,17β-diol werden analog Beispiel 4 g) zu 716 mg 15α-Vinyl-4-androsten-3,17-dion vom Schmelzpunkt 157° C umgesetzt.

$[\alpha]_D$: +117°

b) 530 mg 15α-Vinyl-4-androsten-3,17-dion werden analog Beispiel 4 h) zu 387 mg 4,5-Epoxy-15α-vinylandrostan-3,17-dion umgesetzt.

c) 381 mg 4,5-Epoxy-15α-vinylandrostan-3,17-dion werden analog Beispiel 4 i) zu 207 mg 4-Hydroxy-15α-vinyl-4-androsten-3,17-dion vom Schmelzpunkt 137-138° C umgesetzt.

$[\alpha]_D$: +95,6°

**Beispiel 19**

**15α-(1,2-Diacetoxy-1-butyl)-4-hydroxy-4-androsten-3,17-dion**

a) 1,85 g 15α-But-1-enyl-5-androsten-3β,17β-diol werden analog Beispiel 4 g) zu 1,23 g 15α-But-1-enyl-4-androsten-3,17-dion vom Schmelzpunkt 152-154° C umgesetzt.

b) 1,2 g 15α-But-1-enyl-4-androsten-3,17-dion in 10 ml Tetrahydrofuran werden mit 700 mg N-Methylmorpholin-N-oxid, 2 ml Wasser und 3 ml tert.-Butanol sowie 15 mg Osmiumtetroxid in 2 ml Tetrahydrofuran versetzt und 15 Stunden bei 20° C nachgerührt. Dann wird in schwefelsaures Eiswasser, das 100 mg Natriumsulfid enthält, gegossen, das Produkt abfiltriert, mit Wasser gewaschen und im Vakuum eingeengt. Man erhält 1,3 g rohes 15α-(1,2-Dihydroxy-1-butyl)-4-androsten-3,17-dion.

c) 1,3 g rohes 15α-(1,2-Dihydroxy-1-butyl)-4-androsten-3,17-dion werden analog Beispiel 3 zu 1,1 g 15α-(1,2-Diacetoxy-1-butyl)-4-androsten-3,17-dion vom Schmelzpunkt 143-145° C umgesetzt.

d) 1,1 g 15α-(1,2-Diacetoxy-1-butyl)-4-androsten-3,17-dion werden analog Beispiel 2 c) zu 810 mg 15α-(1,2-Diacetoxy-1-butyl)-4,5-epoxy-androstan-3,17-dion vom Schmelzpunkt 181-183° C umgesetzt.

e) 790 mg 15α-(1,2-Diacetoxy-1-butyl)-4,5-epoxy-androstan-3,17-dion werden analog Beispiel 2 d) zu 412 mg 15α-(1,2-Diacetoxy-1-butyl)-4-hydroxy-4-androsten-3,17-dion vom Schmelzpunkt 158-160°C umgesetzt.
$[\alpha]_D$: -33,4°.

## Patentansprüche

1.) 4,15-Disubstituierte 4-Androsten-3,17-dione der allgemeinen Formel I

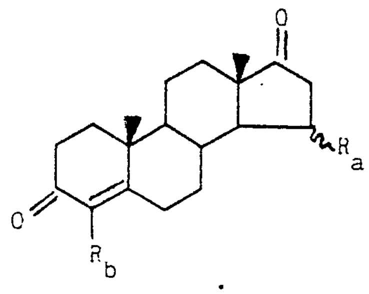

(I) ,

worin
$R_a$ einen gesättigten oder ungesättigten, gegebenenfalls substituierten Alkylrest mit 1-10 Kohlenstoffatomen, wobei $R_a$ in der α- oder β-Position steht,
$R_b$ ein Halogen, eine Azido-, eine $-OR_1$ oder $-NR_2R_3$ Gruppe, wobei $R_1$ ein Wasserstoffatom oder eine Acylgruppe mit 1-10 Kohlenstoffatomen und $R_2$ und $R_3$ gleich oder verschieden sind und jeweils ein Wasserstoffatom, eine Alkyl-oder Acylgruppe mit jeweils 1-10 Kohlenstoffatomen darstellen, bedeuten.

2.) Verbindungen der Formel I nach Anspruch 1, dadurch gekennzeichnet, daß für $R_b$ eine Hydroxyl- oder eine Acyloxygruppe steht.

3.) Verbindungen nach Anspruch 2:
4-Hydroxy-15α-methyl-4-androsten-3,17-dion
15β-Ethyl-4-hydroxy-4-androsten-3,17-dion
4-Acetoxy-15β-ethyl-4-androsten-3,17-dion
15α-Ethyl-4-hydroxy-4-androsten-3,17-dion
4-Acetoxy-15α-ethyl-4-androsten-3,17-dion
4-Hydroxy-15α-hydroxymethyl-4-androsten-3,17-dion
15α-(2-Chlorethyl)-4-acetoxy-4-androsten-3,17-dion
S15α-(1-Acetoxyethyl)-4-hydroxy-4-androsten-3,17-dion
R15α-(1-Acetoxyethyl)-4-hydroxy-4-androsten-3,17dion
4-Hydroxy-15α-propyl-4-androsten-3,17-dion
15α-Butyl-4-hydroxy-4-androsten-3,17-dion
15α-Hexyl-4-hydroxy-4-androsten-3,17-dion
15α-Decyl-4-hydroxy-4-androsten-3,17-dion
4-Hydroxy-15α-vinyl-4-androsten-3,17-dion
15α-(1,2-Diacetoxy-1-butyl)-4-hydroxy-4-androsten-3,17-dion
4-Hydroxy-15α-(2-methylpropyl)-4-androsten-3,17-dion
4-Hydroxy-15α-(3-methylbutyl)-4-androsten-3,17-dion
15α-(Hept-2-yl)-4-hydroxy-4-androsten-3,17-dion
4-Acetoxy-15α-propyl-4-androsten-3,17-dion
4-Acetoxy-15α-butyl-4-androsten-3,17-dion
4-Acetoxy-15α-hexyl-4-androsten-3,17dion
4-Acetoxy-15α-decyl-4-androsten-3,17-dion
4-Acetoxy-15α-(2-methylpropyl)-4-androsten-3,17-dion
4-Acetoxy-15α-(3-methylbutyl)-4-androsten-3,17-dion
15α-Ethyl-4-propionyloxy-4-androsten-3,17-dion
4-Butyryloxy-15α-ethyl-4-androsten-3,17-dion
15α-Ethyl-4-isobutyryloxy-4-androsten-3,17-dion
15α-Ethyl-4-hexanoyloxy-4-androsten-3,17-dion
4-Decanoyloxy-15α-ethyl-4-androsten-3,17-dion

4.) 4-Chlor-15α-ethyl-4-androsten-3,17-dion

5.) Verbindungen der Formel I nach Anspruch 1, dadurch gekennzeichnet, daß für $R_b$ ein stickstoffhaltiger Substituent steht.

6.) Verbindungen nach Anspruch 5:
4-Azido-15α-ethyl-4-androsten-3,17-dion
4-Amino-15α-ethyl-4-androsten-3,17-dion
15α-Ethyl-4-N-methylamino-4-androsten-3,17-dion
4-Acetylamino-15α-ethyl-4-androsten-3,17-dion.

7.) Pharmazeutische Präparate, gekennzeichnet durch den Gehalt an mindestens einer Verbindung gemäß Ansprüche 1-6.

8.) Verwendung der Verbindungen nach mindestens einem der Ansprüche 1-6 zur Herstellung von Präparaten zur Behandlung von östrogen-bedingten Krankheiten.

9.) Verfahren zur Herstellung von 4,15-disubstituierten 4-Androsten-3,17-dionen der allgemeinen Formel 1

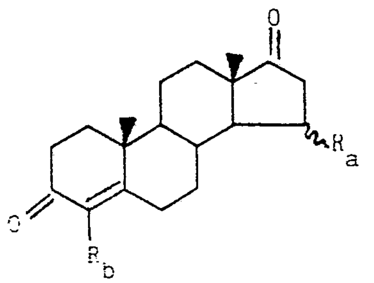

(I) ,

worin
$R_a$ einen gesättigten oder ungesättigten, gegebenenfalls substituierten Alkylrest mit 1-10 Kohlenstoffatomen, wobei $R_a$ in der α- oder β-Position steht,
$R_b$ ein Halogen, eine Azido-, eine $-OR_1$ oder $NR_2R_3$ Gruppe, wobei $R_1$ ein Wasserstoffatom oder eine Acylgruppe mit 1-10 Kohlenstoffatomen und $R_2$ und $R_3$ gleich oder verschieden sind und jeweils ein Wasserstoffatom, eine Alkyl- oder Acylgruppe mit jeweils 1-10 Kohlenstoffatomen, darstellen, bedeuten, dadurch gekennzeichnet, daß man
a) eine Verbindung der allgemeinen Formel II

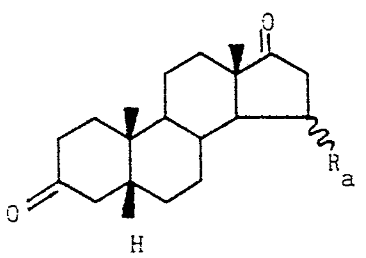

(II) ,

worin
$R_a$ einen gesättigten oder ungesättigten, gegebenenfalls substituierten Alkylrest mit 1-10 Kohlenstoffatomen bedeutet, wobei $R_a$ in der α-oder β-Position steht,
mit Sauerstoff in Gegenwart von Basen oxidiert und das sich gebildete 4-Enol gegebenenfalls verestert, oder
b) ein Epoxid der allgemeinen Formel III,

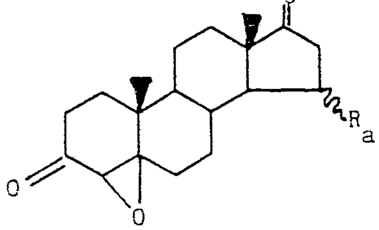

(III) ,

worin
$R_a$ einen gesättigten oder ungesättigten, gegebenenfalls substituierten Alkylrest mit 1-10 Kohlenstoffatomen bedeutet, wobei $R_a$ in der α- oder β-Position steht,
unter protonierenden Bedingungen zur 4-Enolverbindung öffnet und gegebenenfalls verestert, oder mit einem Nucleophil, wie zum Beispiel Chlorid oder Azid, öffnet, dehydratisiert und gegebenenfalls die 4-Azido-Verbindung zur 4-Aminoverbindung hydriert und die Aminogruppe gegebenenfalls alkyliert oder acyliert.

Europäisches Patentamt

# EUROPÄISCHER RECHERCHENBERICHT

Nummer der Anmeldung

EP 88 73 0076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 171 100 (FARMITALIA CARLO ERBA S.p.A.)<br>* Ansprüche *<br>--- | 1,2,7-9 | C 07 J 1/00<br>C 07 J 41/00<br>A 61 K 31/565 |
| X | JOURNAL OF MEDICINAL CHEMISTRY, Band 28, Nr. 6, Juni 1985, Seiten 788-795, American Chemical Society, Washington, DC, US; D.A. MARSH et al.: "Aromatase inhibitors. Synthesis and biological activity of androstenedione derivatives"<br>* Insgesamt *<br>----- | 1,2,7-9 | |

| RECHERCHIERTE SACHGEBIETE (Int. Cl.4) |
|---|
| C 07 J 1/00<br>C 07 J 41/00 |

Der vorliegende Recherchenbericht wurde für alle Patentansprüche erstellt

| Recherchenort | Abschlußdatum der Recherche | Prüfer |
|---|---|---|
| DEN HAAG | 12-07-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)